# EUROPÄISCHE PATENTSCHRIFT

(11) **EP 3 161 478 B1**
(45) Veröffentlichungstag und Bekanntmachung des Hinweises auf die Patenterteilung: **20.06.2018**
(21) Anmeldenummer: 16705016.0
(22) Anmeldetag: 26.01.2016
(51) Int. Cl.: G01N 33/36

(54) **MODULARES GARNPRÜFGERÄT**
MODULAR THREAD TESTING DEVICE
APPAREIL MODULAIRE DE CONTRÔLE DE FIL

(30) Priorität: 20.03.2015 CH 4082015
(43) Veröffentlichungstag der Anmeldung: 03.05.2017
(73) Patentinhaber: Uster Technologies AG, 8610 Uster (CH)
(72) Erfinder: DE VRIES, Loris, 8625 Gossau (CH); FENNER, Jurg, 8600 Dübendorf (CH)
(74) Vertreter: Pliska, Pavel
(86) Internationale Anmeldenummer: PCT/CH2016/000018
(87) Internationale Veröffentlichungsnummer: WO 2016/149840

(56) Entgegenhaltungen:
- CH-A5- 678 764
- GB-A- 2 192 722
- US-A1- 2005 153 649
- "TESTER 5-S800 TECHNICAL DATA", , Dezember 2005 (2005-12), XP55261296, Gefunden im Internet: URL:http://uster.com/fileadmin/customer/Kn owledge/Textile_Know_How/Quality_managemen t/UT5-S800_TechnData-e.pdf [gefunden am 2016-03-29]

## Beschreibung

### FACHGEBIET

Die vorliegende Erfindung liegt auf dem Gebiet der textilen Qualitätsprüfung und bezieht sich auf ein modulares Prüfgerät zur Prüfung von länglichem textilem Prüfgut wie Garn, Vorgarn oder Faserband, gemäss dem Oberbegriff des ersten Patentanspruchs. Das erfindungsgemässe Prüfgerät ist als eigenständige, nicht in einen Produktionsprozess eingebundene Vorrichtung konzipiert und somit im Off-line-Betrieb, bspw. im Textillabor, einsetzbar.

### STAND DER TECHNIK

Prüfgeräte dieser Art werden dort eingesetzt, wo die Qualität von Garnen oder anderen in Faden- oder Bandform vorliegenden textilen Materialien geprüft werden muss. Es geht darum, einerseits eine gleich bleibende Qualität der herzustellenden textilen Produkte zu erreichen, andererseits aber auch darum, den textilen Produktionsprozess anhand von Stichproben off-line zu überwachen.

Geprüft und protokolliert werden mit Prüfgeräten dieser Art in der Regel eine ganze Reihe unterschiedlicher Parameter des zu prüfenden textilen Prüfgutes. Die Anzahl der zu prüfenden Parameter und damit natürlich auch die Komplexität des dazu notwendigen Prüfgerätes hängen von verschiedenen Faktoren ab, so beispielsweise davon, ob Stapel- oder Filamentgarne geprüft werden müssen, aber auch davon, welche Qualitätsstandards für das Textilprodukt vorgegeben sind. Zu den interessierenden Parametern gehört heute in der Regel zumindest die Massenungleichmässigkeit, die mit einem kapazitiven Sensor gemessen wird. Oftmals und gerade bei Naturgarnen kommen aber auch noch optische Sensoren beispielsweise zur Messung von Haarigkeit, Haarigkeitslängen, Fremdstoffe etc. zum Einsatz. Verschiedene weitere interessierende Parameter können dazu treten.

Die bekannten Prüfgeräte dieser Art arbeiten nach dem Durchlaufprinzip, d. h. das zu prüfende Garn wird in einem einzigen Prüfdurchlauf von einer Garnspule abgezogen und durchläuft in serieller Weise im Prüfgerät eine Anordnung von Sensoren, um die zu bestimmenden Parameter zu messen. Dies ist grundsätzlich ein kontinuierlicher Prozess. Je nach Automatisierungsgrad ist auch die mechanische Erfassung und Einführung des Prüfgutes in das Prüfgerät in demselben realisiert. Die Auswertung und statistische Analyse der Sensorsignale erfolgen in der Regel in einer mit dem Prüfgerät verbundenen Steuer- und Auswerteeinheit. Diese interessiert im Kontext der vorliegenden Erfindung jedoch nicht.

Die GB-2' 192'722 A beschreibt eine Vorrichtung zur automatischen Bestimmung von Kenngrössen von textilem Prüfgut wie Garnen, Vorgarnen und Bändern im Durchlaufverfahren. Das eigentliche Prüfgerät umfasst eine Messeinheit zur Gleichmässigkeitsprüfung mit einem Messorgan zur Erfassung von Masseschwankungen, eine Führungseinrichtung und eine Vorschubeinrichtung für das Prüfgut. Dazu kann ein weiteres Messorgan für die Erfassung der Haarigkeit des Prüfgutes hinzutreten. Zwar wird in dieser Offenbarung bereits von einer Modularität gesprochen, es handelt sich dabei aber nicht um Modularität im Sinn eines 'offenen' Systems, sondern lediglich um bis zu maximal drei in der Messeinheit einbaubaren und in unterschiedlicher Reihenfolge zusammenbaubaren Moduleinheiten, die zu einer gemeinsam nutzbaren Grundfunktionalität in der Form einer Fadenführungseinrichtung, einer Vorschubeinrichtung und einer Absaugdüse hinzutreten können. Einrichtungen zum Schutz des Garnpfades und der aus den Moduleinheiten herausragenden Sensoren sind jedoch nicht vorhanden.

Die US-D412290S wie auch die US-D412291S zeigen jeweils ein Garnprüfgerät mit einem turmartigen Aufbau mit einer Mehrzahl gestapelt angeordneter Funktionsmodule. Auch diese Garnprüfgeräte weisen aus den Moduleinheiten herausragende Sensoren auf, allerdings sind hier die einzelnen Moduleinheiten bereits mit Frontabdeckungen versehen, die einen frontseitig vertikal verlaufenden Schlitz für den Garndurchlauf aufweisen. Im Unterschied zur GB-2'192'722 A ist hier also zumindest der Garnpfad besser geschützt, weil er in dem vertikal angeordneten Schlitz verläuft. Bei der US-D412290S ist nicht nur der Garnpfad selber besser geschützt, sondern auch die im Garnpfad angeordneten Sensoren von optionalen Moduleinheiten, die sich oberen Teil des Garnprüfgerätes befinden. Allerdings offenbart die US-D412290S ein Gerät, bei dem eine Garnzuführungseinrichtung so vor den Frontabdeckungen der einzelnen Moduleinheiten angeordnet ist, dass die Frontabdeckungen selber im Falle von Wartungs- und Reparaturarbeiten nur in einem aufwendigen Demontageprozess von den Moduleinheiten entfernt werden können. Die US-2008/0209998 A1 und das Datenblatt "USTER® *TESTER 5-S800"*, Uster Technologies AG, Dezember 2005, zeigen eine Weiterentwicklung des in der US-D412290S offenbarten Garnprüfgerätes.

Die US-5,050,437 A offenbart eine Vorrichtung zur Bestimmung von Festigkeitseigenschaften von langgestrecktem, textilem Prüfgut. Die Vorrichtung besteht aus einem Zuführteil für das zu prüfende Garn, aus einem Speicherteil und aus dem eigentlichen Prüfteil. Jeder dieser Teile kann als Modul ausgebildet sein. Im Prüfteil wird das Garn pneumatisch durch einen Garnkanal gefördert. Der Garnkanal ist durch eine in einer Grundplatte angeordnete Nut oder Rille von U-förmigem Querschnitt gebildet. Der Prüfteil ist durch eine flache Abdeckplatte abgedeckt und abgedichtet, so dass der Garnkanal einen geschlossenen Luftkanal bildet. Die Abdeckplatte kann mittels Scharniere am Prüfteil befestigt sein. Die US-5,050,437 A schlägt vor, die darin offenbarte Festigkeitsprüfvorrichtung mit dem aus der WO-89/03531 A1 bekannten Garngleichmässigkeitsprüfgerät zu einem vollautomatischen, multifunktionalen Prüfgerät zu kombinieren. Das Garngleichmässigkeitsprüfgerät gemäss der WO-89/03531 A1 entspricht im Wesentlichen demjenigen gemäss der oben zitierten GB-2'192'722 A.

Die bekannten Lösungen sind also nicht von der Art, dass sowohl der Schutz des Garnpfades, der Funktionsmodule und der funktionsmoduleigenen Sensoren als auch die Wartbarkeit des Prüfgerätes gleichermassen zufriedenstellend realisiert sind.

### DARSTELLUNG DER ERFINDUNG

Es ist eine Aufgabe der vorliegenden Erfindung, ein modulares Prüfgerät zur Prüfung von länglichem textilem Prüfgut wie Garn, Vorgarn oder Faserband zur Verfügung zu stellen, das die Nachteile des bekannten Standes der Technik überwindet. Das Prüfgerät soll einfach wartbar und reparierbar sein und gleichzeitig einen guten Schutz des Garnpfades, der Funktionsmodule und der funktionsmoduleigenen Sensoren gewährleisten. Der zu erreichende Schutz soll u. a. mechanischen Beschädigungen der Funktionsmodule, Verfälschungen von optischen Messungen durch Fremdlicht von aussen und Verfälschungen der Messungen durch mechanische Eingriffe am Garn entgegenwirken.

Diese und andere Aufgaben werden durch das erfindungsgemässe modulare Prüfgerät, wie es im ersten Patentanspruch definiert ist, gelöst. Vorteilhafte Ausführungsformen sind in den abhängigen Patentansprüchen angegeben.

Der Erfindung liegt die Idee zugrunde, ein modulares Prüfgerät zur Prüfung von faden- und bandartigem Textilgut mit einem turmartigen Aufbau, mit einer Mehrzahl von Funktionsmodulen und mit einer den Funktionsmodulen gemeinsamen Infrastruktur zur Bereitstellung einer Grundfunktionalität so zu gestalten, dass die gemeinsame Infrastruktur mindestens eine mit einem Rahmengehäuse des turmartigen Aufbaus verbundene drehbare oder schwenkbare Frontabdeckung aufweist, die sich über mehrere Funktionsmodule erstreckt. Ein derartiges modulares Prüfgerät beinhaltet zusätzlich eine automatische Einführungsvorrichtung zum Einführen des textilen Prüfgutes in den Prüfgutpfad. Falls eine automatische Einführungsvorrichtung vorhanden ist, sollte sie derart am turmartigen Aufbau angebracht sein, dass die Drehbarkeit oder Schwenkbarkeit der Frontabdeckung nicht beeinträchtigt ist.

Das erfindungsgemässe modulare Prüfgerät dient also zur Prüfung von länglichem textilem Prüfgut wie Garn, Vorgarn oder Faserband. Das Prüfgerät weist einen entlang einer Front des Prüfgerätes verlaufenden Prüfgutpfad, einen turmartigen Aufbau mit einer Mehrzahl entlang des Prüfgutpfades nacheinander angeordneter Funktionsmodule zur Messung verschiedener Parameter des textilen Prüfgutes und mindestens eine Frontabdeckung, die einen frontseitigen, den Prüfgutpfad definierenden Schlitz aufweist, auf. Die mindestens eine Frontabdeckung ist derart am turmartigen Aufbau drehbar befestigt, dass sie durch eine Drehung von einem geschlossenen Zustand, in dem sie mehrere der Funktionsmodule von der Front her zumindest teilweise abdeckt, in einen offenen Zustand, in dem sie diese Funktionsmodule von der Front her zugänglich macht, und wieder zurück überführbar ist.

In einer Ausführungsform weist die mindestens eine Frontabdeckung in einem Bereich mindestens eines der genannten Funktionsmodule eine funktionsmodulspezifische Struktur auf. Die funktionsmodulspezifische Struktur ist z. B. mindestens eine Öffnung oder Ausnehmung für einen Sensor des mindestens einen genannten Funktionsmoduls.

Der frontseitige, den Prüfgutpfad definierende Schlitz kann zumindest in Teilbereichen einen geschlossenen Grund aufweisen.

In einer Ausführungsform ist die mindestens eine Frontabdeckung als für sichtbares Licht undurchlässiges, einstückiges, durch Spritzgiessen hergestelltes Formteil aus Kunststoff ausgeführt.

In einer Ausführungsform weist die mindestens eine Frontabdeckung eine Frontplatte und vier Seitenwände auf, die derart aus den Seiten der Frontplatte hervorragen, dass sie im geschlossenen Zustand Teile der genannten Funktionsmodule von den Seiten her umfassen.

Das Prüfgerät kann genau eine Frontabdeckung beinhalten, welche in geschlossenem Zustand die genannten Funktionsmodule von der Front her vollständig abdeckt.

In einer Ausführungsform weist das erfindungsgemässe Prüfgerät eine automatische Einführungsvorrichtung zum Einführen des textilen Prüfgutes in den Prüfgutpfad auf, welche derart am turmartigen Aufbau angeordnet ist, dass sie die Drehung der mindestens einen Frontabdeckung nicht behindert. Die automatische Einführungsvorrichtung ist vorzugsweise an einer Seite des Prüfgerätes, die von der Front verschieden ist, angeordnet. Die mindestens eine Frontabdeckung kann mittels mindestens eines Scharniers am turmartigen Aufbau drehbar befestigt sein. Das mindestens eine Scharnier befindet sich vorzugsweise an einer Seite des Prüfgerätes, die von der Front und von derjenigen Seite, an der die automatische Einführungsvorrichtung angeordnet ist, verschieden ist.

In einer Ausführungsform liegt eine Achse der Drehung im Wesentlichen parallel zum Prüfgutpfad.

Ein Drehwinkel der Frontabdeckung kann durch eine Begrenzungseinrichtung begrenzt sein, bspw. auf 80° bis 110° und vorzugsweise auf ca. 100°.

In einer Ausführungsform weist das Prüfgerät eine Sicherheitseinrichtung auf, welche sicherheitsrelevante Funktionen des Prüfgerätes nur dann zulässt, wenn die Frontabdeckung vollständig geschlossen ist, und sie ausschaltet, wenn die Frontabdeckung geöffnet ist.

Der Hauptvorteil der Erfindung besteht darin, dass die mehreren Funktionsmodulen gemeinsame türartige drehbare Frontabdeckung für Wartungs-, Reparatur- und Reinigungszwecke sehr viel einfacher zu handhaben ist als Frontabdeckungen für die entsprechenden einzelnen Funktionsmodule. Zudem ist die Frontabdeckung unverlierbar und unverwechselbar am Prüfgerät befestigt. Die angestrebten Schutzfunktionen für Garnpfad, Funktionsmodule und Sensoren bleiben gewahrt.

### AUFZÄHLUNG DER ZEICHNUNGEN

Nachfolgend wird eine Ausführungsform der Erfindung anhand der Zeichnungen detailliert erläutert.
- Figur 1: zeigt ein erfindungsgemässes modulares Prüfgerät in geschlossenem Zustand.
- Figur 2: zeigt das erfindungsgemässe modulare Prüfgerät von Figur 1 in geöffnetem Zustand.

### AUSFÜHRUNG DER ERFINDUNG

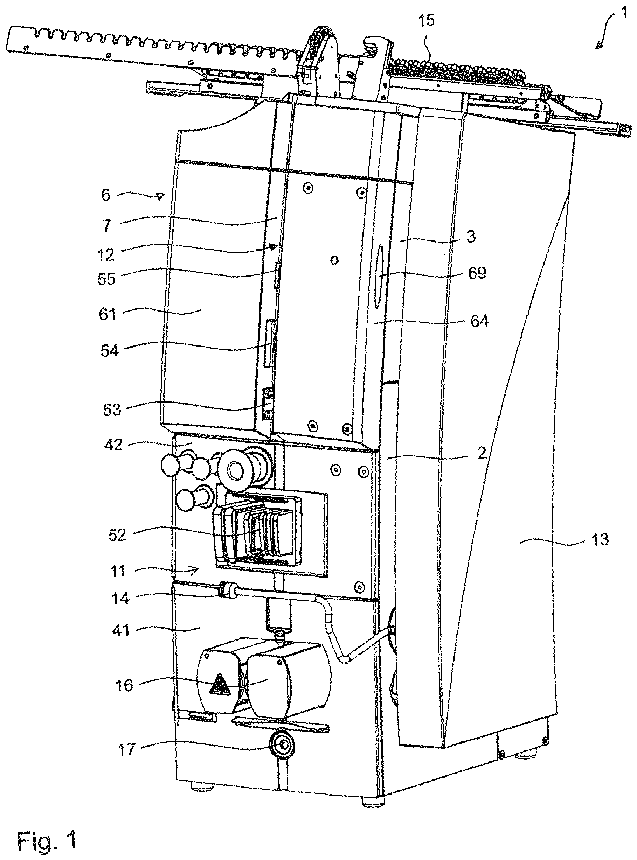

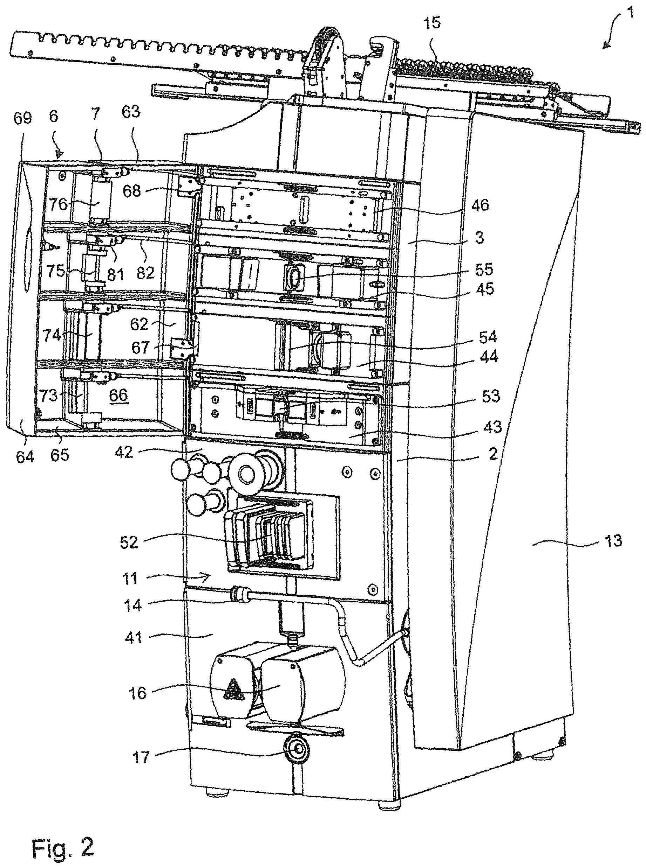

Die Zeichnungen zeigen beispielhaft eine Ausführungsform des erfindungsgemässen modularen Prüfgerätes 1. In der Darstellung von **Figur 1** ist das Prüfgerät 1 geschlossen, während es in **Figur 2** offen ist, wie es bspw. zur Wartung oder Reparatur nötig sein kann.

Das Prüfgerät 1 weist eine Front 11 auf, entlang welcher ein Prüfgutpfad 12 für ein zu prüfendes (nicht eingezeichnetes) textiles Prüfgut verläuft. Auf einer Seite des Prüfgerätes 1, welche von der Front 11 verschieden ist, ist eine automatische Einführungsvorrichtung 13 zum Einführen des Prüfgutes in den Prüfgutpfad 12 angebracht. Zum Einbringen in den Prüfgutpfad 12 wird das Prüfgut von einem verschiebbaren und drehbaren Greifer 14 der Einführungsvorrichtung 13 ergriffen und durch eine entsprechende Bewegung des Greifers 14 eingebracht.

Während des Prüfvorgangs tritt das Prüfgut durch eine automatische Garnwechselvorrichtung 15 in den Prüfgutpfad 12 ein. Im Prüfgutpfad 12 durchläuft das Prüfgut verschiedene Sensoren 52-55, auf die weiter unten eingegangen wird. Das Prüfgut wird von einer Fördereinrichtung 16 entlang seiner Längsrichtung durch den Prüfgutpfad 12 gefördert. Die Fördereinrichtung 16 kann z. B. als Rollenlieferwerk mit zwei zusammenwirkenden Förderrollen von denen mindestens eine zur Drehung angetrieben ist, ausgebildet sein. Schliesslich verlässt das Prüfgut den Prüfgutpfad 12 durch eine Absaugöffnung 17.

Das Prüfgerät 1 hat einen turmartigen Aufbau mit einem Basisgehäuse 2 und einem darauf aufgesetzten Optionengehäuse 3. Das Basisgehäuse 2 und das Optionengehäuse 3 umfassen Funktionsmodule 41-45 zur Prüfung des länglichen Prüfgutes. Jedes Funktionsmodul weist in der Regel einen funktionsmoduleigenen und in den Garnpfad ragenden funktionstypischen Sensor auf. Die einzelnen Sensoren sind meist von unterschiedlicher Grösse, Form und Funktionsweise.

Ein im Basisgehäuse 2 eingebautes erstes Funktionsmodul 41 beinhaltet im vorliegenden Fall die Fördereinrichtung 16 und die Absaugöffnung 17. Ein zweites Funktionsmodul 42 beinhaltet mindestens einen kapazitiven Sensor 52 zur Gleichmässigkeitsprüfung des Prüfgutes. Ein drittes Funktionsmodul 43 beinhaltet einen optischen Mehrzwecksensor 53. An dieser Stelle könnte ebenso gut auch ein anderes der optional einsetzbaren Funktionsmodule 43-46 eingefügt sein.

Auf das Basisgehäuse 2 aufgesetzt und fest mit demselben verbunden ist das Optionengehäuse 3. Das Optionengehäuse 3 bietet im vorliegenden Ausführungsbeispiel Platz für maximal drei Funktionsmodule 44-46, von denen jedes die gleiche Grösse hat wie das im Basisgehäuse 2 eingeschobene dritte Funktionsmodul 43. Das ermöglicht eine wahlfreie Platzierung der optionalen Funktionsmodule 43-46, selbstverständlich nur innerhalb einer messtechnisch sinnvollen Reihenfolge. Ein viertes Funktionsmodul 44 beinhaltet einen optischen Sensor 54 zur Detektion von Verunreinigungen in dem Prüfgut. Ein fünftes Funktionsmodul 45 beinhaltet einen optischen Sensor 55 zur Prüfung der Haarigkeit des Prüfgutes. Ein sechstes Funktionsmodul 46 ist im vorliegenden Ausführungsbeispiel ein Blindmodul, also ein leerer Einschub, der lediglich Platzhalterfunktion hat.

Am turmartigen Aufbau ist eine Frontabdeckung 6 drehbar befestigt. Die Frontabdeckung erstreckt sich über vier Funktionsmodule 43-46, von denen ein Funktionsmodul 43 im Basisgehäuse 2 und die übrigen Funktionsmodule 44-46 im Optionengehäuse 3 eingeschoben sind. Im geschlossenen Zustand, der in Figur 1 dargestellt ist, deckt die Frontabdeckung 6 diese vier Funktionsmodule 43-46 von der Front 11 her ab und schützt sie so vor mechanischen Einwirkungen und Verschmutzung. Im offenen Zustand gemäss Figur 2 ermöglicht sie eine Reinigung, Wartung oder Reparatur der Funktionsmodule 43-46 von der Front 11 her.

Die Frontabdeckung 6 weist eine Frontplatte 61 und vier Seitenwände 62-65 auf. Die Seitenwände 62-65 ragen derart aus den Seiten der Frontplatte 61 hervor, dass sie im geschlossenen Zustand Teile der Funktionsmodule 43-46 von den Seiten her umfassen. Die Frontplatte 61 und die Seitenwände 62-65 zusammen umfassen somit ein im geschlossenen Zustand gegen die Funktionsmodule 43-46 hin gewandtes Innenvolumen 66 von fünf Raumrichtungen her. Die Seitenwände 62-65 verbessern weiter den Schutz vor mechanischen Beschädigungen der Funktionsmodule und vor Verfälschungen von optischen Messungen durch Fremdlicht von aussen.

Die drehbare Befestigung ist im vorliegend diskutierten Ausführungsbeispiel mittels zweier Scharniere 67, 68 gewährleistet, deren Gewerbe an der Frontabdeckung 6 einerseits und an dem Optionengehäuse 3 andererseits befestigt sind. Die Scharniere 67, 68 befinden sich vorzugsweise an derjenigen Seite des Prüfgerätes 1, die von der Front 11 und von derjenigen Seite, an der die automatische Einführungsvorrichtung 13 angeordnet ist, verschieden ist. Eine Achse der Drehung liegt im Wesentlichen parallel zum Prüfgutpfad 12.

Der Drehwinkel der Frontabdeckung 6 ist vorzugsweise durch eine (nicht eingezeichnete) Begrenzungseinrichtung begrenzt, bspw. auf ca. 100°, wie in Figur 2 dargestellt. Geeignete Begrenzungseinrichtungen sind an sich bekannt und könnten z. B. als Gelenk, Kette oder Litze ausgebildet sein.

Die Frontabdeckung 6 weist einen frontseitigen, den Prüfgutpfad 12 definierenden Schlitz 7 auf, der die Frontplatte 61 sowie die obere und untere Seitenwand 63, 65 durchbricht. Im Bereich der Funktionsmodule 43-45 weist die Frontabdeckung 6 funktionsmodulspezifische Strukturen 73-75 auf. Im vorliegend diskutierten Ausführungsbeispiel sind die funktionsmodulspezifischen Strukturen 73-75 im Schlitz 7 angebrachte Öffnungen oder Ausnehmungen 73-75, die an die betreffenden Sensoren 53-55 angepasst sind und ihr Funktionieren ermöglichen. Im Fall der optischen Sensoren 53-55 legen die Öffnungen 73-75 jeweils einen optischen Pfad für das im Sensor verwendete Licht frei, bspw. von einem Lichtsender zum Prüfgut und vom Prüfgut zu einem Lichtempfänger. Auf diese Weise kann auch bei geschlossener Frontabdeckung 6 das durch den Schlitz 7 geführte Prüfgut von den Sensoren 53-55 abgetastet und ausgemessen werden. Bei der Gestaltung des Schlitzes 7 und der Öffnungen 73-75 sollte bedacht werden, dass bei Prüfgeräten 1 dieser Art die Sensoren 52-55 wegen der messverfälschenden Auswirkungen von Verschmutzung und Ablagerung in der Regel belüftet sind. Mit den konkreten Ausformungen des Schlitzes 7 und der Öffnung 73-75 im Bereich eines jeweiligen Sensors 53-55 wird die dortige Belüftungsgüte und damit auch die Messqualität beeinflusst. Statt einfacher Öffnungen 73-75 sind komplexere Formen der funktionsmodulspezifischen Strukturen denkbar, insbesondere dann, wenn Luftströmungen in bestimmte Richtungen gefördert oder unterbunden werden müssen.

Die Frontabdeckung 6 ist vorzugsweise ein für sichtbares Licht undurchlässiges, einstückiges, durch Spritzgiessen hergestelltes Formteil aus Kunststoff. In diesem Formteil können die Öffnungen 73-75 entweder gleich mit eingeformt sein, oder es können Einsätze mit funktionsmodulspezifischen Strukturen für die jeweiligen sensortypische Ausformungen vorgesehen sein. Letzteres ist besonders vorteilhaft, wenn eine wahlfreie Positionierung der optionalen Funktionsmodule 43-46 vorgesehen ist.

Das Prüfgerät 1 kann mit einer (nicht eingezeichneten) Sicherheitseinrichtung ausgestattet sein. Diese lässt sicherheitsrelevante Funktionen des Prüfgerätes nur dann zu, wenn die Frontabdeckung 6 vollständig geschlossen ist, und schaltet sie aus, wenn die Frontabdeckung geöffnet ist. Solche sicherheitsrelevanten Funktionen können z. B. das Leuchten einer Laserlichtquelle in optischen Sensoren 53-55 oder die Bewegung der automatischen Einführungsvorrichtung 13 sein. Die Sicherheitseinrichtung kann z. B. als an einer Innenseite der Frontabdeckung 6 angebrachter Finger ausgebildet sein, der im geschlossenen Zustand (Figur 1) mit einem im turmartigen Aufbau angebrachten Schalter zusammenwirkt.

Die Frontabdeckung 6 kann mindestens eine Griffmulde 68 aufweisen, die einer Bedienperson das Öffnen und Schliessen erleichtert. Die mindestens eine Griffmulde 68 ist vorzugsweise auf einer den Scharnieren 67, 68 gegenüber liegenden Seite der Frontabdeckung angebracht.

In der Frontabdeckung 6 können Blasdüsen 81 (siehe Figur 2) eingebaut sein, denen Druckluft mittels Zuführschläuche 82 zuführbar ist. Die Zuführschläuche 82 sind derart flexibel, dass sie die Drehung der Frontabdeckung 6 ausgleichen können. Durch die aus den Blasdüsen 81 austretenden Teilluftströme werden messverfälschende Faser- und Staubablagerungen an den Sensoren 52-55 und im Garnpfad 12 verhindert. Der Einbau der Blasdüsen 81 in der Frontabdeckung 6 ist gegenüber einem Einbau in den Funktionsmodulen 41-46 vorteilhaft, weil so die Funktionsmodule 41-46 von weiteren Elementen entlastet sind und ihre Wartung einfacher ist.

Zu bemerken ist noch, dass der Schlitz 7 zumindest in Teilbereichen einen geschlossenen Schlitzgrund 76 aufweisen kann. Dies kann beispielsweise dann der Fall sein, wenn das zugehörige Funktionsmodul 46 ein Blindmodul ist.

Selbstverständlich ist die vorliegende Erfindung nicht auf die oben diskutierten Ausführungsformen beschränkt. Bei Kenntnis der Erfindung wird der Fachmann weitere Varianten innerhalb des Schutzumfangs des Anspruchs 1 herleiten können, die auch zum Gegenstand der vorliegenden Erfindung gehören.

### BEZUGSZEICHENLISTE

- 1: Prüfgerät
- 11: Front
- 12: Prüfgutpfad
- 13: Einführungsvorrichtung
- 14: Greifer
- 15: Garnwechselvorrichtung
- 16: Fördereinrichtung
- 17: Absaugöffnung

- 2: Basisgehäuse

- 3: Optionengehäuse

- 41-46: Funktionsmodule

- 52: kapazitive Massesensoren
- 53: optischer Mehrzwecksensor
- 54: optischer Sensor zur Detektion von Verunreinigungen
- 55: optischer Sensor zur Prüfung der Haarigkeit

- 6: Frontabdeckung
- 61: Frontplatte
- 62-65: Seitenwände
- 66: Innenvolumen
- 67,68: Scharniere
- 69: Griffmulde

- 7: Schlitz
- 73-75: Öffnungen
- 76: Schlitzgrund

- 81: Blasdüse
- 82: Zuführschlauch

## Patentansprüche

1. Modulares Prüfgerät (1) zur Prüfung von länglichem textilem Prüfgut wie Garn, Vorgarn oder Faserband, wobei das Prüfgerät (1)
einen entlang einer Front (11) des Prüfgerätes (1) verlaufenden Prüfgutpfad (12),
einen turmartigen Aufbau mit einer Mehrzahl entlang des Prüfgutpfades (12) nacheinander angeordneter Funktionsmodule (41-46) zur Messung verschiedener Parameter des textilen Prüfgutes und
mindestens eine Frontabdeckung (6), die einen frontseitigen, den Prüfgutpfad (12) definierenden Schlitz (7) aufweist,
aufweist,
**dadurch gekennzeichnet, dass**
die mindestens eine Frontabdeckung (6) am turmartigen Aufbau derart drehbar befestigt ist, dass sie durch eine Drehung von einem geschlossenen Zustand, in dem sie mehrere der Funktionsmodule (43-46) von der Front (11) her zumindest teilweise abdeckt, in einen offenen Zustand, in dem sie diese Funktionsmodule (43-46) von der Front (11) her zugänglich macht, und wieder zurück überführbar ist.

2. Modulares Prüfgerät (1) nach Anspruch 1, wobei die mindestens eine Frontabdeckung (6) in einem Bereich mindestens eines der genannten Funktionsmodule (43-46) eine funktionsmodulspezifische Struktur (73-75) aufweist.

3. Modulares Prüfgerät (1) nach Anspruch 2, wobei die funktionsmodulspezifische Struktur (73-75) mindestens eine Öffnung oder Ausnehmung für einen Sensor (53-55) des mindestens einen genannten Funktionsmoduls (43-46) ist.

4. Modulares Prüfgerät (1) nach einem der vorangehenden Ansprüche, wobei der Schlitz (7) zumindest in Teilbereichen einen geschlossenen Grund (76) aufweist.

5. Modulares Prüfgerät (1) nach einem der vorangehenden Ansprüche, wobei die mindestens eine Frontabdeckung (6) als für sichtbares Licht undurchlässiges, einstückiges, durch Spritzgiessen hergestelltes Formteil aus Kunststoff ausgeführt ist.

6. Modulares Prüfgerät (1) nach einem der vorangehenden Ansprüche, wobei die mindestens eine Frontabdeckung (6) eine Frontplatte (61) und vier Seitenwände (62-65) aufweist, die derart aus den Seiten der Frontplatte (61) hervorragen, dass sie im geschlossenen Zustand Teile der genannten Funktionsmodule (43-46) von den Seiten her umfassen.

7. Modulares Prüfgerät (1) nach einem der vorangehenden Ansprüche, wobei das Prüfgerät (1) genau eine Frontabdeckung (6) beinhaltet, welche in geschlossenem Zustand die genannten Funktionsmodule (43-46) von der Front (11) her vollständig abdeckt.

8. Modulares Prüfgerät (1) nach einem der vorangehenden Ansprüche, wobei das Prüfgerät (1) eine automatische Einführungsvorrichtung (13) zum Einführen des textilen Prüfgutes in den Prüfgutpfad (12) aufweist, welche derart am turmartigen Aufbau angeordnet ist, dass sie die Drehung der mindestens einen Frontabdeckung (6) nicht behindert.

9. Modulares Prüfgerät (1) nach Anspruch 8, wobei die automatische Einführungsvorrichtung (13) an einer Seite des Prüfgerätes (1), die von der Front (11) verschieden ist, angeordnet ist.

10. Modulares Prüfgerät (1) nach einem der vorangehenden Ansprüche, wobei die mindestens eine Frontabdeckung (6) mittels mindestens eines Scharniers (67, 68) am turmartigen Aufbau drehbar befestigt ist.

11. Modulares Prüfgerät (1) nach den Ansprüchen 9 und 10, wobei sich das mindestens eine Scharnier (67, 68) an einer Seite des Prüfgerätes (1) befindet, die von der Front (11) und von derjenigen Seite, an der die automatische Einführungsvorrichtung (13) angeordnet ist, verschieden ist.

12. Modulares Prüfgerät (1) nach einem der vorangehenden Ansprüche, wobei eine Achse der Drehung im Wesentlichen parallel zum Prüfgutpfad (12) liegt.

13. Modulares Prüfgerät (1) nach einem der vorangehenden Ansprüche, wobei ein Drehwinkel der Frontabdeckung (6) durch eine Begrenzungseinrichtung begrenzt ist, bspw. auf 80° bis 110° und vorzugsweise auf ca. 100°,

14. Modulares Prüfgerät (1) nach einem der vorangehenden Ansprüche, wobei das Prüfgerät (1) eine Sicherheitseinrichtung aufweist, welche sicherheitsrelevante Funktionen des Prüfgerätes (1) nur dann zulässt, wenn die Frontabdeckung (6) vollständig geschlossen ist, und sie ausschaltet, wenn die Frontabdeckung (6) geöffnet ist.

## Claims

1. A modular tester (1) for testing elongated textile test material such as yarn, roving or sliver, wherein the tester (1) comprises
a test material path (12) extending along a front (11) of the tester (1),
a turret-like setup with a plurality of functional modules (41 to 45) which are arranged in succession along the test material path (12) for measuring various parameters of the textile test material, and
at least one front cover (6) comprising a slot (7) on the front side which defines the test material path (12),
**characterized in that**
the at least one front cover (6) is rotatably fastened to the turret-like setup in such a way that it is transferrable by rotation from a closed state in which it covers a multiple of the functional modules (43 to 46) from the front (11) at least in part to an open state in which it makes said functional modules (43 to 46) accessible from the front (11), and back again.

2. A modular tester (1) according to claim 1, wherein the at least one front cover (6) has a structure (73 to 75) specific to the functional module in a region of at least one of the aforementioned functional modules (43 to 46).

3. A modular tester (1) according to claim 2, wherein the structure (73 to 75) specific to the functional module is at least one opening or recess for a sensor (53 to 55) of the at least one aforementioned functional module (43-46).

4. A modular tester (1) according to one of the preceding claims, wherein the slot (7) has a closed base (76) at least in some sections.

5. A modular tester (1) according to one of the preceding claims, wherein the at least one front cover (6) is formed as an integral shaped part which is made by means of injection moulding from plastic and which is opaque for visible light.

6. A modular tester (1) according to one of the preceding claims, wherein the at least one front cover (6) comprises a front plate (61) and four side walls (62 to 65), which protrude from the sides of the front plate (61) in such a way that in the closed state they enclose parts of the aforementioned functional modules (43 to 46) from the sides.

7. A modular tester (1) according to one of the preceding claims, wherein the tester (1) comprises precisely one front cover (6), which in the closed state fully covers the aforementioned functional modules (43 to 46) from the front (11).

8. A modular tester (1) according to one of the preceding claims, wherein the tester (1) comprises an automatic insertion apparatus (13) for inserting the textile test material into the test material path (12), which is arranged on the turret-like setup in such a way that it does not obstruct the rotation of the at least one front cover (6).

9. A modular tester (1) according to claim 8, wherein the automatic insertion apparatus (13) is arranged on a side of the tester (1) which differs from the front (11).

10. A modular tester (1) according to one of the preceding claims, wherein the at least one front cover (6) is rotatably fastened to the turret-like setup by means of at least one hinge (67, 68).

11. A modular tester (1) according to the claims 9 and 10, wherein the at least one hinge (67, 68) is disposed on a side of the tester (1) which differs from the front (11) and from the side on which the automatic insertion apparatus (13) is arranged.

12. A modular tester (1) according to one of the preceding claims, wherein an axis of rotation lies substantially parallel to the test material path (12).

13. A modular tester (1) according to one of the preceding claims, wherein a rotational angle of the front cover (6) is limited by a limiting device, e.g. to 80° to 110° and preferably to approximately 100°.

14. A modular tester (1) according to one of the preceding claims, wherein the tester (1) comprises a safety device, which only permits safety-relevant functions of the tester (1) when the front cover (6) is completely closed and deactivates them when the front cover (6) is open.

## Revendications

1. Appareil de contrôle modulaire (1) pour le contrôle de matière textile allongée à contrôler telle que du fil, de la mèche ou du ruban de carde, lequel appareil de contrôle (1) présente
un trajet de matière à contrôler (12) passant le long d'une face avant (11) de l'appareil de contrôle (1),
une structure en forme de tour avec plusieurs modules fonctionnels (41-46) disposés les uns à la suite des autres le long du trajet de matière à contrôler (12) pour mesurer différents paramètres de la matière textile à contrôler et
au moins une couverture de face avant (6) qui présente une fente (7) sur l'avant définissant le trajet de matière à contrôler (12),
**caractérisé en ce que**
l'au moins une couverture de face avant (6) est fixée avec possibilité de rotation sur la structure en forme de tour de telle manière qu'elle puisse être amenée par une rotation d'un état fermé, dans lequel elle recouvre au moins en partie plusieurs modules fonctionnels (43-46) à partir de la face avant (11), à une état ouvert, dans lequel elle donne accès à ces modules fonctionnels (43-46) par la face avant (11), et retour.

2. Appareil de contrôle modulaire (1) selon la revendication 1, dans lequel l'au moins une couverture de face avant (6) présente au niveau d'au moins un desdits modules fonctionnels (43-46) une structure spécifique du module fonctionnel (73-75).

3. Appareil de contrôle modulaire (1) selon la revendication 2, dans lequel la structure spécifique du module fonctionnel (73-75) est au moins une ouverture ou un creux pour un capteur (53-55) dudit au moins un module fonctionnel (43-46).

4. Appareil de contrôle modulaire (1) selon l'une des revendications précédentes, dans lequel la fente (7) présente un fond fermé (76) au moins dans certaines zones.

5. Appareil de contrôle modulaire (1) selon l'une des revendications précédentes, dans lequel l'au moins une couverture de face avant (6) est réalisée comme une pièce moulée en plastique non transparente à la lumière visible, d'une seule pièce, fabriquée par moulage par injection.

6. Appareil de contrôle modulaire (1) selon l'une des revendications précédentes, dans lequel l'au moins une couverture de face avant (6) présente une plaque de face avant (61) et quatre parois latérales (62-65) qui dépassent des côtés de la plaque de face avant (61) de façon à entourer par les côtés, dans l'état fermé, des parties desdits modules fonctionnels (43-46).

7. Appareil de contrôle modulaire (1) selon l'une des revendications précédentes, dans lequel l'appareil de contrôle (1) comprend exactement une couverture de face avant (6) qui recouvre complètement lesdits modules fonctionnels (43-46) par la face avant (11) dans l'état fermé.

8. Appareil de contrôle modulaire (1) selon l'une des revendications précédentes, dans lequel l'appareil de contrôle (1) présente un dispositif d'introduction automatique (13) destiné à introduire la matière textile à contrôler dans le trajet de matière à contrôler (12), qui est disposé sur la structure en forme de tour de façon à ne pas faire obstacle à la rotation de l'au moins une couverture de face avant (6).

9. Appareil de contrôle modulaire (1) selon la revendication 8, dans lequel le dispositif d'introduction automatique (13) est disposé sur un côté de l'appareil de contrôle (1) différent de la face avant (11).

10. Appareil de contrôle modulaire (1) selon l'une des revendications précédentes, dans lequel l'au moins une couverture de face avant (6) est fixée avec possibilité de rotation sur la structure en forme de tour au moyen d'au moins une charnière (67, 68).

11. Appareil de contrôle modulaire (1) selon les revendications 9 et 10, dans lequel l'au moins une charnière (67, 68) se trouve sur un côté de l'appareil de contrôle (1) qui est différent de la face avant (11) et du côté sur lequel le dispositif d'introduction automatique (13) est disposé.

12. Appareil de contrôle modulaire (1) selon l'une des revendications précédentes, dans lequel un axe de rotation se trouve sensiblement parallèle au trajet de matière à contrôler (12).

13. Appareil de contrôle modulaire (1) selon l'une des revendications précédentes, dans lequel un angle de rotation de la couverture de face avant (6) est limité par un dispositif de limitation, par exemple entre 80° et 110° et de préférence à environ 100°.

14. Appareil de contrôle modulaire (1) selon l'une des revendications précédentes, dans lequel l'appareil de contrôle (1) comporte une installation de sécurité qui n'autorise les fonctions importantes pour la sécurité de l'appareil de contrôle (1) que lorsque la couverture de face avant (6) est complètement fermée et les désactive quand la couverture de face avant (6) est ouverte.
